# EUROPEAN PATENT SPECIFICATION

(11) **EP 3 479 836 B1**
(45) Date of publication and mention of the grant of the patent: **03.04.2024**
(21) Application number: 17820252.9
(22) Date of filing: 29.06.2017
(51) Int. Cl.: A61K 35/747, A23C 9/12, A23L 33/135, A61K 35/74, A61P 19/02

(54) **CARTILAGE REGENERATION FACILITATING COMPOSITION**
ZUSAMMENSETZUNG ZUR ERLEICHTERUNG DER KNORPELREGENERATION
COMPOSITION FACILITANT LA RÉGÉNÉRATION DU CARTILAGE

(30) Priority: 30.06.2016 JP 2016130847
(43) Date of publication of application: 08.05.2019
(73) Proprietor: Asahi Group Holdings, Ltd., Tokyo 130-8602 (JP); Tokushima University, Tokushima 770-8501 (JP)
(72) Inventor: ROKUTAN Kazuhito, Tokushima-shi Tokushima 770-8501 (JP); NISHIDA Kensei, Tokushima-shi Tokushima 770-8501 (JP); KUWANO Yuki, Tokushima-shi Tokushima 770-8501 (JP); FUJIWARA Shigeru, Sagamihara-shi Kanagawa 252-0206 (JP); SUGAWARA Tomonori, Sagamihara-shi Kanagawa 252-0206 (JP); AOKI Yumeko, Sagamihara-shi Kanagawa 252-0206 (JP); SAWADA Daisuke, Sagamihara-shi Kanagawa 252-0206 (JP)
(74) Representative: D Young & Co LLP
(86) International application number: PCT/JP2017/023850
(87) International publication number: WO 2018/003898

(56) References cited:
- JP-A- 2004 315 477
- JP-A- 2014 113 137
- JP-B2- 5 840 368
- US-A1- 2009 028 840
- US-A1- 2015 283 187
- UTRILLA M.P. et al.: "Probiotic pretreatment diminishes T cell cytokine production in an experimental model of rheumatoid arthritis", Methods Find. Exp. Clin. Pharmacol., vol. 31, no. 6, 2009, page 106, XP009514503,

## Description

### [Technical Field]

The present invention concerns a composition for use in ameliorating joint pain in a subject by promoting cartilage regeneration and a composition for use in ameliorating joint pain in a subject by promoting type 2 collagen synthesis, both compositions comprising, as an active ingredient, lactic acid bacteria according to the invention and/or a treated product thereof according to the invention, wherein the subject suffers from arthritis or is in their 40s or older and has cartilage wear due to ageing.

### [Background Art]

In recent years, locomotive syndrome is increasing due to unbalanced nutritional balance, decreased exercise, decreased muscle strength due to aging, inflammation of joints, and the like. There is a high risk that the locomotive syndrome becomes serious and patients need case. So, prevention and early detection and treatment of exerciser syndrome are regarded as important. Major diseases therefor include osteoarthritis and rheumatoid arthritis. These are caused by inflammation of the joint cartilage and immune abnormality of the joint synovium, and have been treated by controlling inflammation and joint destruction. However, side effects such as adrenal insufficiency and gastrointestinal inflammation have been major problems for steroid preparations and nonsteroidal anti-inflammatory analgesics, which are conventional anti-inflammatory agents.

It has been known as prior arts that the production of type 1 collagen mainly localized on the skin is promoted using lactic acid bacteria, lactic acid bacteria having an anti-inflammatory effect are present, and the collagen degradation is promoted by inflammation. However, there has been no report on the promotion of synthesis of type 2 collagen mainly localized in cartilage and improvement of the synthesis/degradation ratio of type 1 or type 2 collagen.

Specifically, Patent Literature 1 describes the promotion of collagen production by administration of lactic acid bacteria (*Lactobacillus plantarum*)*.* Patent Literature 1 shows in vitro promotion of type 1 collagen production by adding lactic acid bacteria to human cultured cells.

Patent Literature 2 describes the suppression of arthritis by suppressing autoimmune suppression or suppressing inflammation using broken cells of lactic acid bacteria including *Lactobacillus gasseri* strain CP2305, and the improvement of pain therewith.

Patent Literature 3 describes that *Lactobacillus gasseri* strain CP2305 has an effect of activating vagus nerve.

### [Citation List]

### [Patent Literature]

Patent Literature 1: JP Patent Publication No. 2015-096476 A
Patent Literature 2: JP Patent Publication No. 2012-158568 A
Patent Literature 3: JP Patent Publication No. 2012-017282 A

### [Summary of the Invention]

### [Objects to Be Attained by the Invention]

The present invention provides a composition for use in ameliorating joint pain in a subject by promoting cartilage regeneration comprising, as an active ingredient, lactic acid bacteria, wherein the lactic acid bacteria are *Lactobacillus gasseri* strain CP2305 (Accession Number: FERM BP-11331), or *Lactobacillus gasseri* strain CP2305s (Accession Number: NITE BP-1405), and/or a treated product thereof; wherein the treated product thereof comprises disrupted cells or cell-free extract of the lactic acid bacteria; and wherein the subject suffers from arthritis or is in their 40s or older and has cartilage wear due to ageing.

The present invention also provides a composition for use in ameliorating joint pain in a subject by promoting type 2 collagen synthesis comprising, as an active ingredient, lactic acid bacteria, wherein the lactic acid bacteria are *Lactobacillus gasseri* strain CP2305 (Accession Number: FERM BP-11331), or *Lactobacillus gasseri* strain CP2305s (Accession Number: NITE BP-1405), and/or a treated product thereof; wherein the treated product thereof comprises disrupted cells or cell-free extract of the lactic acid bacteria; and wherein the subject suffers from arthritis or is in their 40s or older and has cartilage wear due to ageing.

Described herein, but not part of the invention, is a method that is effective for promoting cartilage regeneration in, in particular, patients suffered from joint pain. Also described herein, but not part of the invention, is a method for promoting type 2 collagen synthesis in, in particular, patients suffered from joint pain.

### [Means for Attaining the Objects]

The present inventors have conducted concentrated studies in order to attain the above objects. As a result, they found that cartilage regeneration could be promoted in a subject to ameliorate joint pain by administering lactic acid bacteria belonging to *Lactobacillus gasseri* according to the invention. Specifically, they found that the type 2 collagen synthesis could be promoted which is higher than degradation of type 2 collagen, and inflammation, pain and stiffness of joints could be alleviated.

### [Effects of the Invention]

Described herein is a composition according to the invention for promoting cartilage regeneration, and a composition according to the invention for promoting type 2 collagen synthesis. The composition of the present disclosure is particularly effective for ameliorating joint pain in a middle-aged subject or a subject suffered from joint pain. In addition, lactic acid bacteria according to the invention as active ingredients are safe and cost-effective, and intake thereof in the form of food or drink products or nutritious supplements can be easy.

### [Brief Description of the Drawings]

Fig. 1 is a graph showing effects of lactic acid bacteria for improving monocytes.
Fig. 2 is a graph showing effects of lactic acid bacteria for improving γ-globulins.
Fig. 3 is a graph showing effects of lactic acid bacteria for improving the type 2 collagen degradation/synthesis ratio (C2C/CPII).
Fig. 4 is a graph showing effects of lactic acid bacteria for improving the Japanese Knee Osteoarthritis Measure (JKOM).
Fig. 5 is a graph showing effects of lactic acid bacteria for improving collagen synthesis indicator (CPII).

### [Embodiments for Carrying out the Invention]

The present invention is based on findings that administration of lactic acid bacteria according to the invention can promote the cartilage regeneration or promote type 2 collagen synthesis, and alleviating inflammation, pain and stiffness of joints, and it can ameliorate joint pain. Specifically, the present invention is based on the finding that administration of lactic acid bacteria *Lactobacillus gasseri* strain CP2305 or *Lactobacillus gasseri* strain CP2305s to the subjects of middle-aged to older people (people in their 40s to 90s) would exert effects of promoting type 2 collagen synthesis and ameliorating joint pain, compared with placebo samples, and the lactic acid bacteria would exert effects of promoting the cartilage regeneration and ameliorating joint pain.

Accordingly, described herein is a composition for promoting cartilage regeneration and a composition for promoting type 2 collagen synthesis comprising, as an active ingredient, lactic acid bacteria according to the invention and/or a treated product thereof according to the invention. In addition, described herein, but not part of the invention, is a method of promoting cartilage regeneration in a subject, and a method of promoting type 2 collagen synthesis in a subject comprising a step of administering lactic acid bacteria and/or a treated product thereof to the subject.

The lactic acid bacteria used in the present invention are capable of producing lactic acid from sugars by fermentation. Bacterial cells of lactic acid bacteria, other than the lactic acid bacteria according to the invention, also known in the art are also described herein but do not form part of the invention, and may exert the function or activity of interest specifically described below. In addition, bacterial strains, other than the lactic acid bacteria according to the invention, that have been confirmed to be safe for animals and which may be preferable in terms of administration to or intake by animals are also described herein but do not form part of the invention.

Other than the *Lactobacillus gasseri* strain CP2305 according to the invention and *Lactobacillus gasseri* strain CP2305s according to the invention, specific examples of other lactic acid bacteria are described herein, but do not form part of the invention, including bacteria belonging to the genus *Lactobacillus*, such as other *Lactobacillus gasseri* strains, *Lactobacillus amylovorus*, *Lactobacillus casei*, *Lactobacillus paracasei*, *Lactobacillus zeae*, *Lactobacillus rhamnosus*, *Lactobacillus reuteri*, *Lactobacillus acidophilus*, *Lactobacillus crispatus*, *Lactobacillus gallinarum*, *Lactobacillus brevis*, *Lactobacillus fermentum*, *Lactobacillus plantarum*, *Lactobacillus delbrueckii subsp. bulgaricus*, and *Lactobacillus johnsonii.*

According to an embodiment, the function or activity of interest may be activity of promoting cartilage regeneration in a subject. According to another embodiment, the function or activity of interest may be activity of promoting type 2 collagen synthesis. The promotion of cartilage regeneration includes the promotion of type 2 collagen synthesis, suppression of type 2 collagen degradation, and both of them. The type 2 collagen synthesis and type 2 collagen degradation can be measured by any method known in the art.

According to the present invention, the function or activity of interest is activity of ameliorating joint pain. In this case, " ameliorating (amelioration of)" joint pain means alleviation of inflammation of joint pain, alleviation of pain and stiffness of joints and removal of cause of joint pain (via cartilage regeneration), and encompasses not only complete removal of joint pain, but also alleviation compared to pre-treatment. Alleviation of inflammation may be determined by, for example, the decrease of blood monocytes or γ-globulins (indicators for inflammation). Pain and stiffness of joints may be evaluated by, for example, the Japanese Knee Osteoarthritis Measure (JKOM). Removal of cause of joint pain may be determined by measuring the promotion of cartilage regeneration, the promotion of type 2 collagen synthesis and/or the suppression of type 2 collagen degradation as described above.

Whether or not a bacterial cell or a treated product of lactic acid bacteria described herein, but not forming part of the invention, exerts the function or activity of interest can be evaluated by administering the bacterial cell or treated product of lactic acid bacteria belonging to the genus *Lactobacillus* to an experimental animal or a test volunteer, and confirming the status of cartilage regeneration before and after the administration, type 2 collagen synthesis, type 2 collagen degradation, amelioration of joint pain and others.

Lactic acid bacteria according to the invention and bacterial cells or a treated product of the lactic acid bacteria evaluated to have the function or activity of interest by the method described above are *Lactobacillus gasseri* strain CP2305 and *Lactobacillus gasseri* strain CP2305s. *Lactobacillus gasseri* strain CP2305 is deposited internationally by the applicant under Accession Number FERM BP-11331 as of September 11, 2007 with the International Patent Organism Depositary, the National Institute of Advanced Industrial Science and Technology (AIST) (Tsukuba Central 6, 1-1-1 Higashi, Tsukuba, Ibaraki, Japan), which is the international depositary authority under the terms of the Budapest Treaty for deposition of patent microorganisms. At present, this strain is transferred to and stored at the Patent Microorganisms Depositary, the National Institute of Technology and Evaluation (120, 2-5-8, Kazusakamatari, Kisarazu-shi, Chiba, Japan), which is the international depositary authority. Also, *Lactobacillus gasseri* strain CP2305s is deposited internationally by the applicant under Accession Number NITE BP-1405 as of August 14, 2012 with the Patent Microorganisms Depositary, the National Institute of Technology and Evaluation (122, 2-5-8, Kazusakamatari, Kisarazu-shi, Chiba, Japan), which is the international depositary authority under the terms of the Budapest Treaty for deposition of patent microorganisms.

*Lactobacillus gasseri* strain CP2305s is a variant obtained from *Lactobacillus gasseri* strain CP2305. Described herein, but not forming part of the invention, are variants of the specific strains mentioned above having the function or activity of interest. For example, variants of the strain CP2305 and the strain CP2305s are highly likely to have the function or activity of interest.

The term "variant" used herein refers to any strain obtained from a parent strain. Specifically, this term refers to a strain obtained by a method of artificially increasing the frequency of mutation from a parent strain by means of spontaneous mutation or mutagenesis with chemical or physical mutagens or a strain obtained by a specific mutagenesis technique (e.g., gene recombination). Microbial individuals resulting from such techniques may be repeatedly subjected to selection and separation, and variants with the function or activity of interest can be obtained by breeding of useful microbial individuals.

For example, variants described herein, but not forming part of the invention, originating from *Lactobacillus gasseri* strain CP2305 or CP2305s can be easily distinguished from other lactic acid bacterial strains based on the molecular weight distribution of amplified genomic DNA fragments of lactic acid bacteria determined by the polymerase chain reaction (PCR). In short, DNA samples of lactic acid bacteria of interest are prepared, gene amplification is carried out by PCR using primers having unique sequence (e.g., 16S rDNA-derived nucleotide sequence), and electrophoresis patterns of the obtained fragments are analyzed. Thus, whether or not the lactic acid bacterial strain of interest is a variant originating from *Lactobacillus gasseri* strain CP2305 or CP2305s can be determined. It should be noted that the technique of confirming whether or not the bacterial strain of interest is a variant is not limited to the above, and whether or not the bacterial strain of interest is a variant can be confirmed by a technique known in the art based on, for example, mycological properties.

Lactic acid bacteria according to the invention can be prepared via culture under adequate conditions using any of media conventionally used for culture of the lactic acid bacteria. In this case, a natural or synthetic medium can be used, as long as it contains a carbon source, a nitrogen source, mineral salts, and the like, and lactic acid bacteria can be cultured efficiently therein. A person skilled in the art can adequately select a known medium suitable for a bacterial strain to be used. Examples of the carbon source that can be used include lactose, glucose, sucrose, fructose, galactose, and blackstrap molasses. Examples of the nitrogen source that can be used include organic nitrogen-containing substances such as casein hydrolysate, whey protein hydrolysate, and soy protein hydrolysate. In addition, examples of the mineral salts that can be used include phosphate, sodium, potassium, and magnesium. Examples of media suitable for culture of lactic acid bacteria include MRS liquid medium, GAM medium, BL medium, Briggs Liver Broth, animal milk, skim milk, and milk-derived whey. Preferably, sterilized MRS medium can be used.

Culture of lactic acid bacteria according to the invention can be performed at 20°C to 50°C, preferably at 25°C to 42°C, and more preferably at approximately 37°C under anaerobic conditions. Temperature conditions can be adjusted using a thermostatic bath, a mantle heater, a jacket, or the like. The term "anaerobic conditions" used herein refers to a low-oxygen environment in which lactic acid bacteria can proliferate. For example, anaerobic conditions can be provided by using an anaerobic chamber, anaerobic box, or airtight container or bag containing a deoxidizer, or by simply sealing a culture container. Examples of culture formats include static culture, shake culture, and tank culture. The period of culture can be 3 hours to 96 hours. It may be preferable to maintain the pH of a medium at 4.0 to 8.0 in the beginning of culture.

Specific examples of lactic acid bacteria preparation are briefly described below. *Lactobacillus gasseri* strain CP2305 or CP2305s lactic acid bacteria may be inoculated into a medium for lactic acid bacteria culture (e.g., an MRS liquid medium) and preferably a food-grade medium for lactic acid bacteria culture, and culture may be carried out overnight (for approximately 18 hours) at approximately 37°C.

After culture, the obtained culture product of the lactic acid bacteria according to the invention can be used in that state, or it may be further subjected to, for example, crude purification via centrifugation and/or solid-liquid separation via filtration and sterilization, according to need. In addition, lactic acid bacteria used in the present invention may be in the form of viable bacterial cells or dead bacterial cells and/or in the form of wet bacterial cells or dried bacterial cells. Use of dead lactic acid bacteria may be preferable.

In addition, a treated product of lactic acid bacteria according to the invention obtained by treating bacterial cells of lactic acid bacteria may be used, as long as it has the function or activity of interest. Alternatively, the treated product of lactic acid bacteria may be further subjected to treatment. Examples of such treatment are described below.

Bacterial cells and/or a treated product of lactic acid bacteria according to the invention can be prepared in the form of a suspension or diluted solution by suspension or dilution in an adequate solvent. Examples of a solvent that can be used include water, physiological saline, and phosphate buffer saline (PBS).

A fermentation product can be prepared by fermenting raw milk, skim milk, or soymilk using bacterial cells and/or a treated product of lactic acid bacteria according to the invention. For example, the lactic acid bacteria or lactic acid bacteria subjected to optional treatment may be inoculated into raw milk, skim milk, or soymilk, followed by fermentation under conditions (substantially equivalent to the above conditions for culture of lactic acid bacteria) known in the art. The thus obtained fermentation product can be used in that state, or it may be subjected to optional treatment such as filtration, sterilization, dilution, or concentration.

A sterilized product of lactic acid bacteria according to the invention can be prepared by sterilization treatment of bacterial cells and/or a treated product of lactic acid bacteria according to the invention. In order to subject bacterial cells and/or a treated product of lactic acid bacteria according to the invention to sterilization treatment, for example, a known technique of sterilization treatment such as filtration sterilization, radiation sterilization, heat sterilization, or high pressure sterilization can be used.

In addition, bacterial cells and/or a treated product of lactic acid bacteria according to the invention can be subjected to heat treatment so as to prepare a heated product of the lactic acid bacteria. In order to prepare such heated product, bacterial cells and/or a treated product of the lactic acid bacteria are(is) subjected to high-temperature treatment (for example, at 80°C to 150°C) for a certain period of time, such as approximately 10 minutes to 1 hour (e.g., approximately 10 to 20 minutes).

Bacterial cells and/or a treated product of lactic acid bacteria according to the invention can be subjected to disruption, fracturing, or grinding to prepare a disrupted product or a cell-free extract. For example, physical disruption (e.g., agitation or filter filtration), enzymatic lysis treatment, chemical treatment, or autolysis treatment can be performed.

Bacterial cells and/or a treated product of lactic acid bacteria according to the invention can be subjected to extraction with the use of an adequate aqueous or organic solvent to obtain an extract. A method of extraction is not particularly limited, as long as such method involves the use of an aqueous or organic solvent as an extraction solvent. For example, a known method, such as a method comprising immersing the lactic acid bacteria or lactic acid bacteria subjected to optional treatment in an aqueous or organic solvent (e.g., water, methanol, or ethanol), or agitating or refluxing the lactic acid bacteria or lactic acid bacteria subjected to optional treatment in the solvent can be adopted.

Also, bacterial cells and/or a treated product of lactic acid bacteria according to the invention can be subjected to drying to process into the form of a powdery product (powder) or granular product. Specific examples of drying methods include, but are not particularly limited to, spray drying, drum drying, vacuum drying, and freeze-drying, which can be used alone or in combination. Upon drying, a conventional excipient may be added, according to need.

In addition, bacterial cells and/or a treated product of lactic acid bacteria according to the invention can be subjected to known separation/purification techniques to purify an ingredient or fraction having the function or activity of interest. Examples of such separation/purification techniques include: a method involving salt precipitation or organic solvent precipitation in accordance with degrees of solubility; a method involving dialysis, ultrafiltration, or gel filtration in accordance with molecular weight differences; a method involving ion-exchange chromatography in accordance with charge differences; a method involving affinity chromatography in accordance with degrees of specific binding; and a method involving hydrophobic chromatography or reversed-phase chromatography in accordance with degrees of hydrophobicity, which can be used alone or in combinations of two or more thereof.

The treatments described above may be carried out alone or in combinations of two or more thereof. Such treated products according to the invention can be used in the composition of the present disclosure.

Through continuous intake of the bacterial cells and/or a treated product of lactic acid bacteria according to the invention obtained above alone or in combination with other ingredients in the form of the composition of the present disclosure, food or drink products, feeds, nutritious supplements, or pharmaceutical compositions, effects of promoting cartilage regeneration and/or promoting type 2 collagen synthesis, and effects of ameliorating joint pain attained by the effects mentioned above can be contemplated.

The composition according to the invention comprises, as an active ingredient, the bacterial cells and/or a treated product of lactic acid bacteria according to the invention. Such composition may comprise a single type of bacterial cells and/or a treated product of lactic acid bacteria according to the invention, a plurality of different types of bacterial cells and/or a treated product of lactic acid bacteria according to the invention, or treated products of a plurality of lactic acid bacteria according to the invention subjected to different treatments in combination.

In addition to the bacterial cells and/or a treated product of lactic acid bacteria according to the invention as active ingredients, the composition of the present disclosure may be supplemented with the additives described below, other known drugs, or nutritious supplements alone or in combinations of two or more, as long as the function or activity of interest is not inhibited.

While the dosage form of the composition of the present disclosure is not particularly limited, examples of dosage forms include: oral formulations such as tablets, capsules, granules, powders, dust formulations, syrups, dry syrups, solutions, suspensions, and inhalers; enteral formulations such as suppositories; infusions; and parenteral injections. Among them, an oral formulation may be preferable. In addition, a liquid formulation, such as a solution or suspension, may be dissolved or suspended in water or a different adequate medium immediately before use. In the case of tablets or granules, surfaces thereof may be coated by a well-known method. Further, the composition of the present disclosure may be prepared in the form of a controlled-release formulation, such as a sustained-release formulation, a delayed-release formulation, or an immediate-release formulation, by a technique known in the art.

The composition in the dosage form described above can be prepared in accordance with a conventional method by formulating conventional additives, such as excipients, disintegrants, binders, wetting agents, stabilizers, buffering agents, lubricants, preservatives, surfactants, sweeteners, flavoring agents, aromatics, acidulants, and coloring agents, into the ingredients described above in accordance with the dosage form. When the composition of the present disclosure is prepared in the form of a pharmaceutical composition, for example, a pharmaceutically acceptable carrier or additive can be incorporated into the composition. Examples of such pharmaceutically acceptable carriers and additives include water, pharmaceutically acceptable organic solvents, collagen, polyvinyl alcohol, polyvinyl pyrrolidone, carboxyvinyl polymers, sodium alginate, water-soluble dextran, water-soluble dextrin, sodium carboxymethyl starch, pectin, xanthan gum, gum Arabic, casein, gelatin, agar, glycerin, propylene glycol, polyethylene glycol, vaseline, paraffin, stearyl alcohol, stearic acid, human serum albumin, mannitol, sorbitol, lactose, surfactants acceptable as pharmaceutical additives, and artificial cell constructs such as liposomes.

When the composition of the present disclosure comprises the additives described above or other drugs, the content of the bacterial cells and/or a treated product of lactic acid bacteria according to the invention as active ingredients varies depending on the dosage form. The content of the lactic acid bacteria may be generally 0.0001% to 99% by mass, preferably 0.001% to 80% by mass, and more preferably 0.001% to 75% by mass. It may be preferable that the composition be prepared into a dosage form that allows management of the daily dose, so as to achieve intake of the active ingredients in preferable amounts. In addition, the number of the lactic acid bacteria or a treated product thereof to be incorporated into the composition of the present disclosure may be approximately 10⁷ cells/g to approximately 10¹² cells/g (when counted as the number of bacterial cells of lactic acid bacteria before treatment).

Examples of other drugs that can be added to or incorporated into the composition of the present disclosure include, but are not limited to, chondroitin, vitamin B1, glucosamine, and hyaluronic acid.

The composition of the present disclosure may further contain a variety of additives used for production of medicaments, food or drink products, and feeds and other various substances. Examples of such substances and additives include a variety of fats and oils (e.g., plant oils, such as soybean oil, corn oil, safflower oil, and olive oil, and animal fats and oils, such as beef fat and sardine oil), crude drugs (e.g., royal jelly and ginseng), amino acids (e.g., glutamine, cysteine, leucine, and arginine), polyalcohols (e.g., ethylene glycol, polyethylene glycol, propylene glycol, glycerin, and sugar alcohols such as sorbitol, erythritol, xylitol, maltitol, and mannitol), natural polymers (e.g., gum Arabic, agar, water-soluble corn fibers, gelatin, xanthan gum, casein, gluten or gluten hydrolysate, lecithin, starch, and dextrin), vitamins (e.g., vitamin C and B-complex vitamins), minerals (e.g., calcium, magnesium, zinc, and iron), dietary fibers (e.g., mannan, pectin, and hemicellulose), surfactants (e.g., glycerin fatty acid esters and sorbitan fatty acid esters), purified water, excipients (e.g., glucose, cornstarch, lactose, and dextrin), stabilizers, pH-adjusting agents, antioxidants, sweeteners, taste components, acidulants, coloring agents, and flavors.

The amount of such additive can be adequately determined depending on the type of additive and the desirable amount to be intaken. The content of bacterial cells and/or a treated product of lactic acid bacteria according to the invention as active ingredients may vary depending on the dosage form. The content may be generally 0.0001% to 99% by mass, preferably 0.001% to 80% by mass, and more preferably 0.001% to 75% by mass, as the amount of the lactic acid bacteria before treatment.

Subjects of administration or intake of the composition of the present disclosure may be vertebrate animals. Specific examples thereof include mammals such as humans, primates (e.g., monkeys and chimpanzees), livestock animals (e.g., cattle, horses, pigs, sheep, and chickens), pet animals (e.g., dogs and cats), and experimental animals (e.g., mice and rats). Further, subjects can be reptiles and birds (e.g., chickens). Particularly preferable subjects may be those for whom joint pains are expected to be ameliorated, such as human subjects suffered from arthritis, and middle-aged to older people having cartilage wear due to aging (human subjects in their 40s or older).

The dose of administration or intake of the composition of the present disclosure may vary depending on the age and the body weight of a subject, the administration/intake route, the administration/intake frequency, the purpose of administration (e.g., promotion of cartilage regeneration, promotion of type 2 collagen, amelioration of joint pain), and other conditions. The dose of administration or intake can be changed extensively at the discretion of a person skilled in the art to achieve a desired effect. For oral administration or intake, for example, it may be preferable to administer bacterial cells and/or a treated product of lactic acid bacteria according to the invention contained in the composition according to the invention in an amount of generally approximately 10⁶ cells to 10¹² cells, and preferably approximately 10⁷ cells to 10¹¹ cells per kg of body weight, as the amount of the bacterial cells of lactic acid bacteria. The content of bacterial cells and/or a treated product of lactic acid bacteria according to the invention is not particularly limited and can be adequately adjusted in accordance with the ease of production, the preferable daily dose, or other conditions. Since the composition of the present disclosure is highly safe, it is also possible to further increase the dose to be administered. A daily dose may be administered in a single instance or in several separate instances. In addition, the frequency of administration or intake is not particularly limited, and it can be adequately selected depending on various conditions, such as the route of administration or intake, age or body weight of a subject, and desired effects (e.g., promotion of cartilage regeneration, promotion of type 2 collagen, or amelioration of joint pain).

The administration or intake route of the composition of the present disclosure is not particularly limited, and it can be, for example, oral administration or intake or parenteral administration (e.g., intrarectal, subcutaneous, intramuscular, or intravenous administration). Particularly preferably, the composition of the present disclosure may be orally administered or ingested.

The composition of the present disclosure improves the cartilage regeneration, and promotes the type 2 collagen synthesis. In particular, the composition may be effective for joint pains. Specifically, the type 2 collagen synthesis may be promoted, inflammation may be suppressed, and joint pains may be ameliorated in the subject, compared with a control to which the composition of the present disclosure (lactic acid bacteria according to the invention and/or a treated product thereof) is not administered. Thus, the composition of the present disclosure has effects of ameliorating joint pains in a subject, in particular, a human subject suffered from arthritis and middle-aged or older subject having cartilage wear due to aging (human subjects in their 40s or older).

The composition of the present disclosure may be used in combination with an other medicament or an other therapeutic or preventive method. The "other medicament" and the composition of the present disclosure may be formulated into a single formulation. Alternatively, they may be formulated into separate formulations to administer them simultaneously or at intervals.

As described above, the composition of the present disclosure can be used in the form of a pharmaceutical composition for promoting cartilage regeneration, promoting type 2 collagen synthesis, ameliorating joint pains, and other purposes.

In addition, the composition of the present disclosure is highly safe and thus is easily used for long-term continuous intake. Therefore, the composition of the present disclosure can also be added to food or drink products, nutritious supplements, or feeds. The composition of the present disclosure has the function or activity described above, and it contains lactic acid bacteria according to the invention that have been used for meals. Thus, such composition is highly safe. Further, even when it is added to a variety of food or drink products, it does not inhibit the flavor of a food or drink product itself. Thus, it can be added to various types of food or drink products and continuously ingested. As a consequence, the function or activity of interest can be expected.

The food or drink product described herein contains the composition of the present disclosure as described above. The food or drink product described herein also includes beverages. Examples of the food or drink product containing the composition of the present disclosure include functional food or drink products aimed at better health by functions or activity, including promotion of cartilage regeneration, promotion of type 2 collagen synthesis, and amelioration of joint pain (e.g., nutritious supplements, food for specified health use, foods with nutrient function claims, and foods with functional claims), and all food or drink products into which the composition of the present disclosure can be incorporated.

Functional food or drink products are particularly preferable as food or drink products containing the composition of the present disclosure. The "functional food or drink product" described herein means a food or drink product having a predetermined function for organisms and encompasses, for example, all of so-called health food or drink products such as food or drink products with health claims including foods for specified health use (FOSHU) and food or drink products with nutrient function claims, foods with function claims, foods for special dietary uses, nutritional supplements, health supplements, supplements (e.g., those having a variety of dosage forms such as tablets, coated tablets, sugar-coated tablets, capsules, and liquid agents), and beauty food or drink products (e.g., diet food or drink products). The functional food or drink products described herein also encompass health food or drink products to which health claim based on Codex (Joint FAO/WHO Food Standards Programme) food standards are applied.

Specific examples of food or drink products include: health food or drink products and nutritional supplements in preparation forms such as liquid diets (e.g., tube enteral nutritional supplements), tablet candies, tablets, chewable tablets, tablets, dust formulations, powders, capsules, granules, and tonic drinks; tea beverages such as green tea, oolong tea, and black tea; beverage products such as soft drinks, jelly beverages, sport beverages, milk beverages, carbonated beverages, vegetable beverages, juice beverages, fermented vegetable beverages, fermented fruit juice beverages, fermented milk beverages (e.g., yogurt), lactic acid bacteria beverages, milk beverages (e.g., coffee milk and fruit milk), beverages containing drink powders (e.g., milk formula), cocoa beverages, milk, and purified water; spreads such as butter, jam, dried seasoning products, and margarine; mayonnaise; shortening; custard; dressings; breads; boiled rice; noodles; pasta; Japanese *miso* soup; Japanese *tofu*; yogurt; baby foods; soups or sauces; and sweets (e.g., biscuits and cookies, chocolates, candies, cakes, ice creams, chewing gums, and tablets).

The food or drink product described herein can be produced by conventional methods by adding other food materials used for production of the above-mentioned food or drink products, various nutrients, various vitamins, minerals, dietary fibers, and various additives (e.g., taste components, sweeteners, acidulants such as organic acids, stabilizers, and flavors), in addition to the above-mentioned active ingredients.

For the food or drink product described herein, a person skilled in the art can adequately determine the amount of the lactic acid bacteria according to the invention and/or a treated product thereof as an active ingredient to be incorporated thereinto by taking the form of the food or drink product and the desirable taste or texture into consideration. In general, a person skilled in the art may appropriately determine the total amount of bacterial cells and/or a treated product of lactic acid bacteria according to the invention to be added to the composition, so as to adjust the amount of lactic acid bacteria to 0.0001% to 99% by mass, preferably 0.001% to 80% by mass, and more preferably 0.001% to 75% by mass. The composition of the present disclosure is highly safe. As such, the amount of the composition incorporated into a food or drink product can be further increased. In order to achieve consumption of the desirable amount of the composition, it may be desirable to prepare the composition in a dosage form that allows management of the daily dose. As described above, the food or drink product described herein can be consumed in a manner that allows management of a desirable amount of the composition of the present disclosure. Thus, a method for promoting cartilage regeneration, a method for promoting type 2 collagen synthesis, and a method for ameliorating joint pain using such food or drink products is described herein but is not part of the invention.

The composition of the present disclosure may be incorporated into a food or drink product by any appropriate method available to a person skilled in the art. For example, the composition of the present disclosure can be prepared in the liquid, gel, solid, powdery, or granular form and the resultant is then incorporated into the food or drink product. Alternatively, the composition of the present disclosure may be mixed or dissolved directly into raw materials of the food or drink product. The composition of the present disclosure may be applied to, coated onto, infiltrated into, or sprayed onto the food or drink product. The composition of the present disclosure may be dispersed uniformly or distributed unevenly in the food or drink product. A capsule or the like containing the composition of the present disclosure may be prepared. The composition of the present disclosure may be wrapped with an edible film or a food coating agent. Alternatively, an adequate excipient or the like may be incorporated into the composition of the present disclosure, and the resultant may be prepared in the form of, for example, a tablet. The food or drink product containing the composition of the present disclosure may further be processed. Such a processed product also falls within the scope of the present invention.

The food or drink product described herein may be prepared with the use of various types of additives that are commonly used for food or drink products.

As described above, the food or drink product described herein has useful effects, it is highly safe, and thus there is no concern about side effects. Further, the composition of the present disclosure has a favorable flavor. Even when it is added to a variety of food or drink products, it does not inhibit the flavor of the food or drink product. Accordingly, the resulting food or drink product can be easily subjected to long-term continuous ingestion, and useful effects thereof can be expected for a long period of time.

Further, the composition of the present disclosure can be formulated not only into food or drink products for humans but also into feeds for animals such as livestock (e.g., cattle, pigs, and chickens), racehorses, and pets (e.g., dogs and cats). Feeds are substantially equivalent to food or drink products except that they are given to non-human subjects. Therefore, the descriptions concerning food or drink products above can also be applied to feeds.

Hereafter, the present invention is described in greater detail with reference to the examples, although the present invention is not limited to these examples.

### [Test Example]

### (1) Preparation of lactic acid bacteria

As lactic acid bacteria, *Lactobacillus gasseri* strain CP2305 (Accession Number: FERM BP-11331) and *Lactobacillus gasseri* strain CP2305s (Accession Number: NITE BP-1405) were prepared.

### (2) Measurement of monocytes and γ-globulins

Blood was collected from subjects, and the number of monocytes was measured by a flow cytometry method. Using the blood serum, γ-globulins (immunoglobulins Ig-G, Ig-A and Ig-M) were measured by an immunoturbidimetric method.

### (3) Measurement of the ratio of type 2 collagen degradation/synthesis (C2C/CPII)

Using the blood serum from the subjects and using ELISA, C2C was measured by using "Collagen Type II Cleavage Assay" of IBEX Technilogies Inc. and CPII was measured by using "type II collagen C-terminal propeptide (PIICP) enzyme-linked immunosorbent assay kit" of USCN. The ratio of the measured values was calculated to obtain C2C/CPII.

### (4) Measurement of the Japanese Knee Osteoarthritis Measure (JKOM)

The Japanese Knee Osteoarthritis Measure (JKOM) was determined by summarizing the results filled out by the subjects on the questionnaire of the Japanese Knee Osteoarthritis Measure (JKOM).

### [Example 1]

### <Preparation of test beverage>

With the use of *Lactobacillus gasseri* strain CP2305 and *Lactobacillus helveticus* strain, skimmed milk powder and an yeast extract were fermented at 32°C to 37°C for 12 to 22 hours, the resulting fermented milk was supplemented with liquid sugar, a sweetener, an acidulant, a stabilizer, and a flavoring agent, and the resultant was then sterilized.

A placebo beverage was prepared and sterilized in the same manner as described above with the use of milk fermented with *Lactobacillus helveticus* strain under the same conditions while refraining from the use of the strain CP2305.

A paper bottle was filled with 200 ml of the sterilized milk, and the test beverage and the placebo beverage were prepared.

### [Example 2]

### <Test of beverage for human use>

Subjects (34 subjects, middle-aged to older people in their 40s to 90s) were divided into two groups, a bottle of the test beverage containing *Lactobacillus gasseri* strain CP2305 (the amount of bacterial cells: 10¹⁰ cells) or the placebo beverage that does not contain the strain CP2305 was administered every day over the period of 12 weeks.

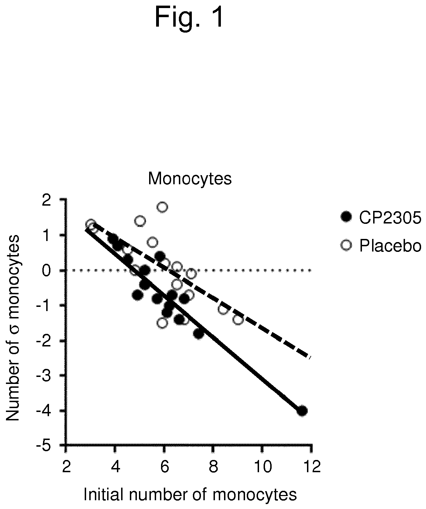

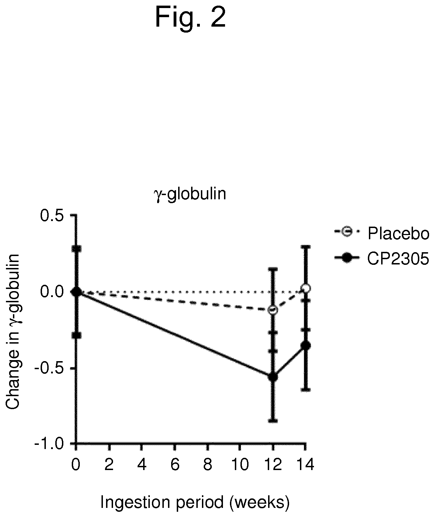

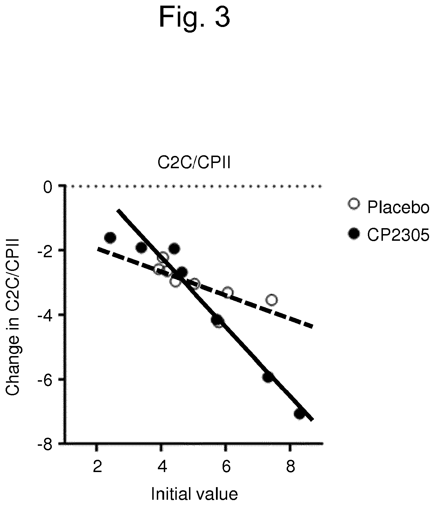

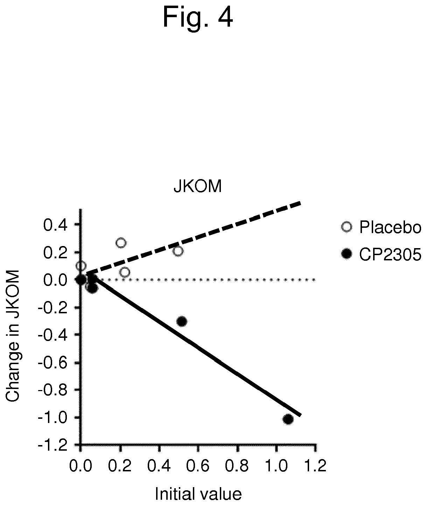

As a result, decreases in the monocytes and γ-globulins, blood indicators for inflammation, were observed in the group to which the strain CP2305 had been administered (Figs. 1 and 2). The decrease in the type 2 collagen degradation/synthesis ratio (C2C/CPII) was also observed (Fig. 3), and demonstrating that the type 2 collagen synthesis was promoted compared to the type 2 collagen degradation. Fig. 1 shows the plot of changes in the number of monocytes relative to the initial number and the results of calculation of the regression lines with the least square method. Fig. 2 shows the changes in the γ-globulins relative to a period of drinking (weeks). Fig. 3 shows the plot of changes in the indicator, the type 2 collagen degradation/synthesis ratio (C2C/CPII) relative to the initial value and the results of calculation of the regression lines with the least square method. The decrease in the score value using the Japanese Knee Osteoarthritis Measure (JKOM) was also observed in the group to which the strain CP2305 had been administered (Fig. 4). Fig. 4 shows the plot of changes relative to the initial scores of the Japanese Knee Osteoarthritis Measure (JKOM) and the results of calculation of the regression lines with the least square method. These results demonstrate the effects of ameliorating joint pains of the knees in subjects, especially in the middle-aged to older subjects.

### [Example 3]

### <Test of beverage for human use>

Subjects (18 subjects, older people in their 70s to 90s) were subjected to the test similarly to that of Example 2, and the changes in a collagen synthesis indicator, CPII before and after the administration were determined.

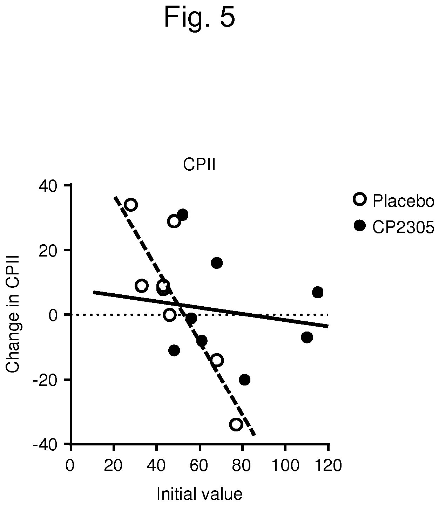

The results are shown in Fig. 5. The suppression of the decrease of CPII, a collagen synthesis indicator, was observed in the group to which the strain CP2305 had been administered (Fig. 5). Accordingly, the collagen synthesis is significantly promoted and maintained in the group to which the strain CP2305 had been administered when compared to the placebo group.

### [Accession Numbers]

Accession Number: FERM BP-11331 (*Lactobacillus gasseri* strain CP2305; date of deposition: September 11, 2007)
Accession Number: NITE BP-1405 (*Lactobacillus gasseri* strain CP2305s; date of deposition: August 14, 2012)

## Claims

1. A composition for use in ameliorating joint pain in a subject by promoting cartilage regeneration comprising, as an active ingredient, lactic acid bacteria, wherein the lactic acid bacteria are *Lactobacillus gasseri* strain CP2305 (Accession Number: FERM BP-11331), or *Lactobacillus gasseri* strain CP2305s (Accession Number: NITE BP-1405), and/or a treated product thereof; wherein the treated product thereof comprises disrupted cells or cell-free extract of the lactic acid bacteria; and wherein the subject suffers from arthritis or is in their 40s or older and has cartilage wear due to ageing.

2. A composition for use in ameliorating joint pain in a subject by promoting type 2 collagen synthesis comprising, as an active ingredient, lactic acid bacteria, wherein the lactic acid bacteria are *Lactobacillus gasseri* strain CP2305 (Accession Number: FERM BP-11331), or *Lactobacillus gasseri* strain CP2305s (Accession Number: NITE BP-1405), and/or a treated product thereof; wherein the treated product thereof comprises disrupted cells or cell-free extract of the lactic acid bacteria; and wherein the subject suffers from arthritis or is in their 40s or older and has cartilage wear due to ageing.

3. The composition for use according to claim 1 or 2, wherein the promotion of cartilage regeneration or the promotion of type 2 collagen synthesis is indicated by the decrease in the collagen degradation/synthesis ratio (C2C/CPII).

4. The composition for use according to any one of claims 1 to 3, which is selected from the group consisting of food or drink products, nutritious supplements, and medicaments.

5. The composition for use according to claim 4, wherein the food or drink products comprise fermented milk beverages, yogurt, powdered milk, baby foods, *miso* soup, retort foods, and tablets.

6. The composition for use according to claims 1 to 5, wherein the subject is a human subject in their 40s or older.

## Patentansprüche

1. Zusammensetzung zur Verwendung bei der Linderung von Gelenkschmerzen bei einem Patienten durch Förderung der Knorpelregeneration, die als Wirkstoff Milchsäurebakterien, wobei es sich bei den Milchsäurebakterien um *Lactobacillus gasseri*-Stamm CP2305 (Zugangsnummer: FERM BP-11331) oder *Lactobacillus gasseri*-Stamm CP2305s (Zugangsnummer: NITE BP-1405) handelt, und/oder ein behandeltes Produkt davon umfasst, wobei das behandelte Produkt davon aufgebrochene Zellen oder zellfreien Extrakt der Milchsäurebakterien umfasst und wobei das Subjekt an Arthritis leidet oder in den 40er Jahren oder älter ist und an altersbedingten Knorpelverschleiß leidet.

2. Zusammensetzung zur Verwendung bei der Linderung von Gelenkschmerzen bei einem Subjekt durch Förderung der Synthese von Typ-2-Kollagen, die als Wirkstoff Milchsäurebakterien, wobei es sich bei den Milchsäurebakterien um *Lactobacillus gasseri*-Stamm CP2305 (Zugangsnummer: FERM BP-11331) oder *Lactobacillus gasseri*-Stamm CP2305s (Zugangsnummer: NITE BP-1405) handelt, und/oder ein behandeltes Produkt davon umfasst, wobei das behandelte Produkt davon aufgebrochene Zellen oder zellfreien Extrakt der Milchsäurebakterien umfasst und wobei das Subjekt an Arthritis leidet oder in den 40er Jahren oder älter ist und an altersbedingten Knorpelverschleiß leidet.

3. Zusammensetzung zur Verwendung nach Anspruch 1 oder 2, wobei die Förderung der Knorpelregeneration oder die Förderung der Synthese von Typ-2-Kollagen durch die Abnahme des Abbau/Synthese-Verhältnisses von Kollagen (C2C/CPII) angezeigt wird.

4. Zusammensetzung zur Verwendung nach einem der Ansprüche 1 bis 3, ausgewählt aus der aus Lebensmittel- oder Getränkeprodukten, Nahrungsergänzungsmitteln und Arzneimitteln bestehenden Gruppe.

5. Zusammensetzung zur Verwendung nach Anspruch 4, wobei die Lebensmittel- oder Getränkeprodukte fermentierte Milchgetränke, Joghurt, Milchpulver, Babynahrung, Misosuppe, Retortenlebensmittel und Tabletten umfassen.

6. Zusammensetzung zur Verwendung nach einem der Ansprüche 1 bis 5, wobei es sich bei dem Subjekt um ein menschliches Subjekt in den 40er Jahren oder älter handelt.

## Revendications

1. Composition pour une utilisation dans l'amélioration d'une douleur articulaire chez un sujet en favorisant la régénération de cartilage comprenant, en tant qu'un ingrédient actif, des bactéries d'acide lactique, les bactéries d'acide lactique étant une souche de *Lactobacillus gasseri* CP2305 (numéro d'accès : FERM BP-11331), ou une souche de *Lactobacillus gasseri* CP2305S (numéro d'accès : NITE BP-1405), et/ou un produit traité correspondant ; le produit traité correspondant comprenant des cellules perturbées ou un extrait sans cellules des bactéries d'acide lactique ; et le sujet souffrant d'arthrite ou étant d'un âge de 40 ans ou plus et présentant une usure du cartilage due au vieillissement.

2. Composition pour une utilisation dans l'amélioration d'une douleur articulaire chez un sujet en favorisant la synthèse de collagène de type 2 comprenant, en tant qu'un ingrédient actif, des bactéries d'acide lactique, des bactéries d'acide lactique étant une souche de *Lactobacillus gasseri* CP2305 (numéro d'accès : FERM BP-11331), ou une souche de *Lactobacillus gasseri* CP2305S (numéro d'accès : NITE BP-1405), et/ou un produit traité correspondant ; le produit traité correspondant comprenant des cellules perturbées ou un extrait sans cellules des bactéries d'acide lactique ; et le sujet souffrant d'arthrite ou étant d'un âge de 40 ans ou plus et possédant une usure du cartilage due au vieillissement.

3. Composition pour une utilisation selon la revendication 1 ou 2, la favorisation de la régénération de cartilage ou la favorisation de la synthèse de collagène de type 2 étant indiquée par la diminution du rapport dégradation/synthèse de collagène (C2C/CPII).

4. Composition pour une utilisation selon l'une quelconque des revendications 1 à 3, qui est choisie dans le groupe constitué par des produits alimentaires ou à boire, des suppléments nutritionnels et des médicaments.

5. Composition pour une utilisation selon la revendication 4, les produits alimentaires ou à boire comprenant des boissons au lait fermenté, un yaourt, un lait en poudre, des aliments pour bébés, une soupe *miso*, des aliments en poche de cornue et des comprimés.

6. Composition pour une utilisation selon les revendications 1 à 5, le sujet étant un sujet humain d'un âge de 40 ans ou plus.
